# EUROPÄISCHE PATENTSCHRIFT

(11) **EP 0 859 957 B1**
(45) Veröffentlichungstag und Bekanntmachung des Hinweises auf die Patenterteilung: **03.04.2002**
(21) Anmeldenummer: 96945812.4
(22) Anmeldetag: 03.10.1996
(51) Int. Cl.: G01N 33/543, G01N 33/548, G01N 33/564

(54) **PATIENTENSPEZIFISCHE IMMUNADSORBER FÜR DIE EXTRAKORPORALE APHERESE UND VERFAHREN FÜR DEREN HERSTELLUNG**
PATIENT-SPECIFIC IMMUNOADSORBENTS FOR EXTRACORPORAL APHERESIS AND METHODS OF PRODUCING SAID IMMUNO-ADSORBERS
IMMUNO-ADSORBANTS SPECIFIQUES A CHAQUE PATIENT POUR UNE APHERESE EXTRACORPORELLE ET PROCEDE DE FABRICATION CORRESPONDANT

(30) Priorität: 05.10.1995 DE 19538641
(43) Veröffentlichungstag der Anmeldung: 26.08.1998
(73) Patentinhaber: Privates Institut Bioserv GmbH, 18059 Rostock (DE)
(72) Erfinder: HEINRICH, Hans-Werner, D-17498 Riemserort (DE); RAMLOW, Wolfgang, D-18209 Bad Doberan (DE); BOEDEN, Hans-Friedrich, D-13187 Berlin (DE); NEUMANN, Hans-Georg, D-18146 Rostock (DE); MEYER, Udo, D-18239 Hastorf (DE); TELLER, Joachim, D-18273 Güstrow (DE)
(74) Vertreter: Baumbach, Friedrich, Dr.
(86) Internationale Anmeldenummer: DE9601910
(87) Internationale Veröffentlichungsnummer: WO9714964

(56) Entgegenhaltungen:
- WO-A-86/07152
- US-A- 4 551 435
- CLINICAL AND EXPERIMENTAL IMMUNOLOGY, Bd. 53, 1983, OXFORD UK, Seiten 529-535, XP000670721 C. BRUNEAU ET AL.: "Evidence for a disease specific antigen in circulating immune complexes in ankylosing spondylitis"

## Beschreibung

Die Erfindung betrifft die Herstellung von Immunadsorbern auf der Grundlage der spezifischen Antikörper und/oder Antigene, die in Folge von immunpathologischen Prozessen für die Auslösung oder Aufrechterhaltung vieler Erkrankungen verantwortlich sind. Dadurch wird es möglich, gezielt in den immunpathologischen Regulationskreis einzugreifen, der für die klinischen Folgen verantwortlich ist, ohne das gesamte Immunsystem in Mitleidenschaft zu ziehen, wie es z.B. bei der herkömmlichen Therapie durch medikamentöse Immunsuppression der Fall ist.

Darüber hinaus ermöglicht das erfindungsgemäße Verfahren die Anreicherung und Reindarstellung der immunpathologisch relevanten körpereigenen Stoffe und eröffnet damit neue Möglichkeiten für die Ursachenforschung und Therapieentwicklung.

Die funktionelle Grundlage für die Immunreaktion ist komplex und beruht auf dem gut regulierten Zusammenwirken von lokalen und systemisch wirksamen zellulären und humoralen Elementen der unspezifischen Abwehr und dem System der spezifischen Abwehr, bestehend aus aktivierten Zellen des lymphopoetischen Systems und den von ihnen produzierten Mediatoren und Antikörpern.

Je nach der Art der Stimulierung können die vorherrschenden Abwehrleistungen unterschiedlich sein.

Diese Steigerung der Abwehrlage wird auf natürliche Weise im Verlauf eines Infektionsgeschehens oder künstlich, d.h. medikamentös, erworben. Das erfolgt sowohl systemisch als auch lokal über die Schleimhäute des Respirations-, Digestions- und Urogenitaltraktes.

Natürlicherweise reagiert der Organismus sofort auf eine Infektion. Die Qualität und Quantität der Sofortreaktion hängt ab von der Art des Antigens und dem Ort des Eindringens. Prinzipiell gleiche Reaktionen erfolgen bei Verabreichung von Impfstoffen und anderen körperfremden Substanzen. Eine spezifische Abwehr, meßbar z.B. durch den Nachweis spezifischer Antikörper, ist erst nach einigen Tagen wirksam. Ist die auslösende Ursache beseitigt, wird die Produktion spezifischer Antikörper nach unten reguliert und letztlich eingestellt. Nach erfolgtem biologischen Abbau der Antikörper weisen nur noch das Vorhandensein spezifischer "Gedächtniszellen" darauf hin, mit welchen Antigenen sich der Organismus in der Vergangenheit auseinander gesetzt hat. Unter bestimmten Umständen, im einzelnen meist nicht mehr bestimmbarer Kausalität, reagiert der Organismus in überschießender Weise auf körpereigene Strukturen. Die sich entwikkelnde Autoimmunreaktion führt zur fortlaufenden Destruktion körpereigenen Gewebes, deren Abbauprodukte wiederum das Immunsystem stimulieren. Wird dieser pathologische Regelkreis nicht unterbrochen, sind die Folgen letztlich zumindest für das betroffene Gewebe fatal.

Erkrankungen immunpathologischer Genese oder Beteiligung sind häufig. Durch ihren chronischen Verlauf und ihre schwere Therapierbarkeit beeinträchtigen sie die Lebensqualität der betroffenen Menschen erheblich und verursachen enorme volkswirtschaftliche Verluste. Eine der häufigsten Autoimmunkrankheiten ist die Rheumatoide Arthritis, an der etwa 1 % der Bevölkerung erkrankt. Das Hauptmanifestationsalter der Erkrankung liegt um das 40. Lebensjahr. Nach 10 Jahren sind etwa 50 % der Patienten erwerbsunfähig und 10 - 20 % schwerstbehindert. Die bislang erreichten Behandlungsergebnisse durch Immunsuppression und unterstützende Therapien sind unzureichend und enden häufig in Therapieabbrüchen. Nach 3 Jahren sind maximal nur noch 50 % der initial mit Basistherapeutika behandelten Patienten unter effektiver Medikation.

Wegen der häufig unzureichenden Wirksamkeit und großer Nebenwirkungen herkömmlicher immunsuppressiver Therapie wird ständig nach neuen Therapieverfahren zur Behandlung von Autoimmunerkrankungen gesucht (J.Sany: Early approaches to immunotherapy of rheumatoid arthritis. EUR-J-RHEUMATOL-INFLAM: 11(1991), 139 - 147).

Diese Therapien zielen auf die Beeinflussung von humoralen und zellulären Immunmechanismen wie auch von Mediator-Systemen. Es handelt sich dabei um experimentelle Therapieansätze, die im Tierversuch und in der klinischen Erprobung erste Erfolge brachte. Ein entscheidender Durchbruch in der Prognose von Patienten mit Autoimmunkrankheiten konnte allerdings bislang nicht erzielt werden.

Plasmaaustausch und Plasmasorption sind erfolgreich bei einer Vielzahl von Autoimmunkrankheiten und Erkrankungen immunpathologischer Beteiligung eingesetzt worden (R.T. Baldwin, R. R. Pierce und O. H. Frazier: Guillain-Barre syndrome after heart transplantation. J-HEART-LUNGTRANSPLAN: 11 (1992), 817-819; J. Braun, J. Sieper, A. Schwarz, F. Keller, J. Heitz und H.V. Ameln: Severe lupus crisis with agranulocytosis and anuric renal failure due to a mesangial lesion (WHO IIB)-Successful treatment with cyclophosphamide pulse followed by plasmapheresis (2). BR-J-RHEUMATOL: 30 (1991), 312-313; P. C. Dau: Plasmapheresis in acute multiple sclerosis: Rationale and results. J-CLIN-APHERESIS: 6 (1991), 200-204; H. H. Euler, J. O. Schroeder, R. A. Zeuner und E. Teske: A randomized trial of plasmapheresis and subsequent pulse cyclophosphamide in severe lupus: Design of the LPSG trial. INT-J-ARTIF-ORGANS: 14 (1991), 639-646; D. C. Hess, K. Sethi und E. Awad: Thrombotic thrombocytopenic purpura in systemic lupus erythematosus and antiphospholipid antibodies: Effective treatment with plasma exchange and immunosuppression. J-RHEUMATOL: 19 (1992), 1474-1478; R. Korinthenberg und M. Sauer: The Guillain-Barre syndrome in childhood. Clinical course and therapeutic measures. MONATSSCHR-KINDERHEILKD: 140 (1992), 792-798)

Der Plasmaaustausch ist das ältere Therapieverfahren, bei dem separiertes Plasma (Membranplasmapherese oder Zentrifugation) verworfen und simultan und durch Spenderplasma oder Humanalbumin ersetzt wird. Während einer Behandlung wird die einfache bis doppelte Menge des Patientenplasma ausgetauscht. Dieses Verfahren ist nicht selektiv. Um einen oder wenige pathogenetisch wichtige Bestandteile zu entfernen, wird das gesamte Plasma ausgetauscht und Substanzen verworfen, die für den Patienten essentiell sind. Das hat schwerwiegende Folgen für den Patienten, die man versucht mit verschiedenen Substitutionstherapien auszugleichen. Daneben besteht die Gefahr der Übertragung von Krankheitserregern wie das HIV oder Hepatitiserreger.

Bei der Plasmasorption wird das zuvor separierte Plasma über Adsorbermaterial geleitet. An das Adsorbermaterial sind Stoffe gekoppelt, die mit bestimmten Plasmabestandteilen eine Bindung eingehen und diese dadurch aus dem Patientenplasma entfernen. Wird die Plasmasorption zum Entfernen von immunologisch bedeutsamen Substanzen genutzt, wird das Verfahren als Immunadsorption bezeichnet. Das Verfahren besitzt in Abhängigkeit vom verwendeten Adsorbermaterial eine unterschiedliche Selektivität und Spezifität. Diese wiederum bedingt Effektivität der Therapie und Nebenwirkungen. Unterschiedliche Liganden und Träger sind zur Adsorption von Immunglobulinen und Immunkomplexen aus dem separierten Plasma klinisch eingesetzt worden:

### Tabelle 1:

### Beispiele von Liganden mit klinischer Anwendung in extrakorporalen Apherese-Verfahren

- Staphylokokken-Protein A
- hydrophobe Aminosäuren (Tryptophan bzw. Phenylalanin)
- Dextransulfat
- aggregiertes IgG
- anti-Human-IgG
- Blutgruppen-Antigene

Die folgende Tabelle gibt einen Überblick über erfolgreich mit der extrakorporalen Immunadsorption behandelte Autoimmunerkrankungen.

### Tabelle 2:

Autoimmunerkrankungen mit erfolgreicher Apherese-/Immunadsorptions-Behandlung
- rapid-progressive Glomerulonephretis, fokale Glomerulosklerose
- systemischer Lupus erythermatodes
- Anti-Phospholipid-Syndrom
- Vaskulititiden; z. B. Periarteriitis nodosa, M. Wegener
- Rheumatoid-Arthritis
- immunologische thrombozytopenische Purpura
- Hemmkörper gegen Koagulatiosfaktoren
- hyperimmunisierte bzw. ABO-inkompatible prospektive Transplantat-Empfänger
- Polymyoisitis
- neurologische Erkrankungen; z.B. Guillain-Barre-Syndrom, Polyneuropathie, amyotrophe Lateralsklerose, Myasthenia gravis, Multiple Sklerose

Nachteil der medikamentösen Therapien bei Autoimmunerkrankungen Die medikamentöse Immunsuppression ist unselektiv und unspezifisch. Auch neuartige immunologische Therapien (monoklonale oder polyklonale Antikörper gegen Aktivierungsmarker oder Rezeptorstrukturen von Immunzellen und Mediatoren) supprimieren die Immunantwort nicht selektiv und/oder induzieren ihrerseits Immunphänomene im Organismus.

### Nachteile von bisherigen Apherese/-Adsorptionsverfahren in der Behandlung von Autoimmunerkrankungen

Der Nachteil aller bisher bekannten Apherese-/Adsorptionssysteme besteht analog zur medikamentösen Immunsuppresion in ihrer unzureichenden Selektivität. Das gilt für das bereits angewendete Verfahren von Balint und Hargreavens (US-Patent 4.681.870), die Staphylococcus-aureus-Protein A an geeignete Träger immobilisierten. Mit dieser Methode werden aus dem Patientenblut unspezifisch IgG und IgG-Komplexe entzogen. Das trifft auch auf das von Davis (PCT-Anmeldung WO 86/07152) beschriebene Verfahren der Nutzung von trägergekoppelten unspezifischen Proteinen, vorzugsweise Immunglobuline verschiedener Spezies, als Immunadsorbents von Immunkomplexen zu. Mittels dieser Methode werden wohl Immunkomplexe aus dem eleminiert, nicht aber die reaktiven Einzelbestandteile, die bei Autoimmunkrankheiten ständig neu entstehen.

Liberti und Pollora (US-Patent 4.551.435) beschreiben ein Verfahren zur Entfernung von Stoffen und Immunkomplexen aus dem Blut, indem das Patientenblut mit spezifischen Antikörpern einer bestimmten Konzentration versetzt wird, die dann mit der zu eliminierenden Substanz Immunkomplexe bilden. Diese werden aus dem Blut mit Faktoren wie C1q, Rheumafaktoren, Fc-Rezeptoren und Fc-Rezeptoren-tragenden Zellen, die an einen festen Träger immobilisiert sind, aus dem Blut eliminiert. Dieses Verfahren setzt voraus, daß die auslösende Ursache bekannt ist, was bei den meisten Fällen von Autoimmunkrankheiten nicht der Fall ist und daß das ursächliche Antigen für die Herstellung von Antikörpern gereinigt vorliegt. Die Entfernung der Immunkomplexe selbst erfolgt unspezifisch nicht über Protein A, sondern durch Biomoleküle, die physiologischerweise eine hohe Affinität zu Immunglobulinen haben.

Es gilt zu beachten, daß die pathophysiologisch relevanten Immunstrukturen bei den einzelnen Autoimmunerkrankungen verschieden sind, selbst die Immunphänomene bei ein- und derselben Erkrankung. Der Einsatz bislang verfügbarer Apherese-Systeme führt nicht nur zur Eliminierung der immunpathologisch bedeutsamen sondern auch der physiologischen - für die körpereigene Abwehr essentiellen - Immunglobuline. Das Resultat ist eine generelle Schwächung des Immunsystems mit der Gefahr septischer Komplikationen.

C. BRUNEAU el al. (1983), Clin. exp. Immunol 53, 529-535 beschreiben Immunadsorber, bei denen Proteine auf einer Plastikoberfläche adsorbiert werden.

Die Erfindung hat das Ziel, eine Therapie für Patienten zur Verfügung zu stellen, die an Krankheiten leiden, die durch Dysregulation des Immunsystems bedingt sind oder die sich durch immunpathologische Prozesse zu chronischen, schwer therapierbaren Verlaufsformen entwickeln. Ihr liegt die Aufgabe zugrunde, einen für den jeweiligen Patienten spezifischen Immunadsorber zu entwickeln, mit dessen Hilfe die pathogenetisch bedeutsamen Immunkomplexe, Autoantikörper und Antigene durch Adsorption aus dem Blut oder Plasma des Patienten entfernt werden können.

Diese Aufgabe wird gemäß den Ansprüchen 1 und 7 gelöst, die Unteransprüche sind Vorzugsvarianten.

Dazu werden die Immunkomplexe mit bekannten Verfahren, z.B. mittels Protein A - Immunadsorbern, aus dem Plasma des Patienten entfernt und nach entsprechender Elution in ihre biologisch aktiven Bestandteile zerlegt. Jetzt können die Bestandteile durch bekannte Verfahren, z.B. Gel-Chromatographie, getrennt und einzeln, bzw. als Gemisch von Antikörpern und Antigenen an entsprechendes Trägermaterial gekoppelt werden. Mit Hilfe dieser Immunadsorber können aus dem Plasma des Patienten spezifisch die krankheitsbedeutenden Immunkomplexe, Antikörper und Antigene mittels Plasmapherese entfernt werden. Diese Säulen sind reaktivierbar und zum mehrfachen Gebrauch vorgesehen. Die Herstellung solcher patientenspezifischer Immunadsorber ist generell für jede Krankheit möglich, wo Autoimmunkomplexe eine pathogenetische Rolle spielen.

Die Lösung dieser Aufgaben ergibt sich aus den Patentansprücheh.

Der patientenspezifische Immunadsorber besteht gemäß der Erfindung aus getrennten Antigenen und/oder Antikörpern aus Immunkomplexen pathologisch relevanter Immunfaktoren von Patienten, die an aktivierte feste Trägermaterialien kovalent gebunden sind. Er enthält getrennte Antigene und/oder Antikörper von Patienten, die an Erkrankungen leiden, die durch Dysregulation hervorgerufen oder aufrechterhalten werden. Autoimmunkrankheiten oder immunpathologische Reaktionslagen sind u.a.

Rheumatoid-Arthritis, rapid-progressive Glomerulonephritis, systemischer Lupus erythematosus, Antiphosphoid-Syndrom, Vaskulitiden, histoinkompatiblen Transplantatempfängern, Polymyositis, neurologische Autoimmunkrankheiten oder immunpathologische Dysregulationen in Folge von Infektionskrankheiten. Geeignet als Trägermaterialien sind alle bioverträglichen Stoffe, die ausreichend viele Immunkomplexbestandteile auf ihrer Oberfläche kovalent binden können; bevorzugt ist der Einsatz von Sepharose und Perlcellulose.

Das Verfahren zur Herstellung der patientenspezifischen Immunadsorber ist durch folgende Schritte gekennzeichnet:
Immunkomplexe werden zunächst mit bekannten, nicht selektiven Verfahren, z.B. mittels Protein A - Immunadsorbern, aus dem Plasma der Patienten entfernt und nach entsprechender Elution in ihre biologisch aktiven Bestandteile zerlegt. Jetzt können die Bestandteile durch bekannte Verfahren, z.B. Gel-Chromatographie, getrennt oder einzeln, bzw. als Gemisch von Antikörpern und Antigen an entsprechendes Trägermaterial gekoppelt werden. Bevorzugt ist die Zerlegung der Immunkomplexe im sauren bzw. alkalischen Milieu, vorzugsweise in pH-Bereichen von 2-5 bzw. 10-12 in ihre Einzelbestandteile, die ggf. nach Fraktionierung und ggf. nach Zusatz von Salzen wie NaCl, MgCl₂, LiCl oder Harnstoff oder Guanidinghydrochlorid, die geeignet sind, ab einer bestimmten Konzentration die Reaktionspartner im dissoziierten Zustand zu halten, in einem pH-Bereich von 2-12 mittels an sich bekannter Methoden an die festen Materialien gekoppelt werden.

Mit Hilfe dieser Immunadsorber können aus dem Plasma des Patienten spezifisch 'seine', die krankheitsbedeutenden Immunkomplexe, Antikörper und Antigene mittels extrakorporaler Immunadsorption entfernt werden. Diese Säulen sind reaktivierbar und zum mehrfachen Gebrauch vorgesehen. Die Herstellung solcher patientenspezifischer Immunadsorber ist generell für jede Krankheit möglich, wo Autoimmunkomplexe eine pathogenetische Rolle spielen.

Das Verfahren ermöglicht neben der therapeutischen Anwendung die Isolierung von Stoffen aus dem Patientenblut, die für die Auslösung der immunologischen Dysregulation zumindest mitverantwortlich sind. Das vereinfacht Untersuchungen zur Pathogenese von Autoimmunkrankheiten bzw. von Erkrankungen, die durch immunologische Fehlleistungen in ihrem Verlauf verstärkt werden. Der Vorteil gegenüber herkömmlichen Lösungen ist:
1. Es werden nicht nur die Immunkomplexe entfernt, sondern auch die bisher nicht erfaßten einzelnen Reaktionspartner.
2. Eine Substitution mit Fremd-Immunglobulinen ist nicht mehr notwendig (Krankheitsübertragung wie HIV werden ausgeschlossen, zusätzliche Kosten vermieden).
3. Ohne Wissen der Krankheitsursache können kostengünstig patientenspezifische Immunadsorber hergestellt werden. Damit können spezifische Therapiewerkzeuge auch für solche Autoimmunkrankheiten bereitgestellt werden, für die wegen der geringen Erkrankungshäufigkeit gezielte Entwicklungen durch die Industrie aus Kostengründen abgelehnt werden.
4. Es können die Antigene und/oder Antikörper spezifisch angereichert, isoliert und damit für weitere Untersuchungen bereitgestellt werden, die für die Auslösung bzw. Aufrechterhaltung der Autoimmunkrankheit des einzelnen Patienten verantwortlich sind.

Im folgenden wird die Erfindung näher erläutert.

Patienten, die an Autoimmunkrankheiten wie z.B. Rheumatoid-Arthritis, Lupus erythematodes oder Multibler Sclerose leiden, werden einer extrakorporalen Apherese unter Verwendung von Staphylokokken-Protein A-Immunadsorbern unterzogen. Nach Beendigung eines Apheresezyklus wird die Säule gründlich mit einem Puffer gewaschen, dem Detergenzien zugesetzt sind, oder die durch eine erhöhte Ionenkonzentration (z.B. 1 - 3 mol/l NaCl) adsorptiv gebundene Plasmabestandteile von der Säule entfernen. Das Freisein von adsorptiven Plasmabestandteilen wird durch Elektrophorese oder Immunassay des Spülpuffers überprüft. Anschließend erfolgt die Elution der Immunglobuline, der Immunkomplexe und der dissoziierten immunologischen Reaktionspartner mittels eines pH-Gradienten (z.B. Citrat oder Acetatpuffer pH 7 - 2) oder konzentrierten Salzlösungen unterschiedlichen pH-Wertes (zwischen 4 und 7). Mittels Elektrophorese, Chromatographie oder anderen geeigneten Trennverfahren werden die eluierten Fraktionen hinsichtlich ihres Proteinspektrums und Dissoziationsgrades von Immunkomplexen untersucht.

Für die Immobilisierung an feste Träger werden Fraktionen verwendet, in denen die Immunkomplexe in ihre reaktiven Bestandteile aufgespalten sind. Diese können vor der Kopplung bei Bedarf durch geeignete Trennverfahren separiert werden. Die Immunkomplexbestandteile werden einzeln oder als Gemisch mittels bekannter Verfahren an ONB-Cabonat- oder N-Hydroxy-succinimid-Ethylester aktvierte Trägermaterialen gekoppelt. Nach Entfernen aller nicht gebundenen Bestandteile steht ein patientenspezifischer und regenerierbarer Immunadsorber zur Verfügung, mit dessen Hilfe selektiv aus dem Patientenblut nur die Stoffe entfernt werden, die für die immunpathologische humorale Dysregulation verantwortlich sind.

Immunkomplexe können aus Blut oder anderen Körperflüssigkeiten, wie z.B. Liquor cerebrospinalis isoliert werden.

Anschließend wird die Erfindung an Ausführungsbeispielen näher erläutert.

### Ausführungsbeispiel 1

Modelluntersuchungen zur Bestimmung der biologischen Aktivität von immobilisierten humanen IgG durch Bindung von anti-human IgG (Ziege) - Tabelle 3
Die Kopplung des humanen IgG an die jeweiligen Träger (Sepharose 6FF und Perlcellulose) erfolgte unter Elutionsbedingungen. Cl-CO-ONB-aktiviertes Gel mit ca. 30 µmol ONB-Carbonatgruppen pro ml wurde für die Kopplung des humanen IgG verwendet.

1 ml Antiserum (5,3 mg anti-human IgG) wurde mit 1 ml PBS verdünnt und über die jeweiligen Träger gegeben (Flußrate von 0,1 ml / min). Die Säulen wurden mit mehreren Säulenvolumen PBS und 3 M NaCl pH 5,0 gewaschen. Die Elution erfolgte mit 0,1 M Glycin-HCl, 0,05 % Tween® 20, pH 2,0 (Flußrate 1,0 ml / min, 2 - 6 °C). Die Bestimmung der Proteinkonzentration erfolgte spektro-photometrisch bei einer Wellenlänge von 280 nm nach Neutralisation der Eluate mit 0,5 M K₂HPO₄. Die relative Antikörperbindungskapazität pro ml Gel ist bezogen auf die Kopplung von humanen IgG unter Standardbedingungen (0,5 M Phosphatpuffer, 0,05 % Tween® 20, pH 7,2).

### Ausführungsbeispiel 2

Modelluntersuchungen zur Bestimmung der Bindungskapazität von humanen IgG (Antigen), immobilisiert mittels Basen-aktivierte ONB-Carbonat Sepharose® 6FF bei pH 3,0, durch Affinitätschromatographie unter Verwendung von anti-human IgG (Antikörper) im Überschuß - Tabelle 4

Für die Kopplung wurde humanes IgG (Sigma) im Kopplungspuffer gelöst und filtriert (0,2 µm). Diese Lösung wurde der lösungsmittelfeuchten aktivierten Sepharose® zugesetzt. Die Kopplung erfolgte bei Raumtemperatur (1 h) unter vorsichtiger Bewegung. Nach Blockung (1 M Ethanolamin in 0,1 M Borat, pH 8,0) für 1 h wurde das Gel intensiv gewaschen, z.B. an einer Fritte mit je dem 10-fachen des Säulenvolumens in der Abfolge: Kopplungspuffer - Wasser - 0,01 N Hcl - Wasser - 24 h 0,1 Boratpuffer, pH 8,3 - Wasser.

Die Affinitätschromatographie erfolgte bei 2 - 6 °C mit dem ECONO-System (Bio-Rad) unter Verwendung der Säule Omnifit® 5,0 x 0,3 cm I.D. (350 µl Gel). Es wurden Flußraten wurden zwischen 0,25 und 1,0 ml/min gewählt. Die Elution wurde mittels UV-Durchflußphotometer (280 nm) vermessen. Nach der Antikörperbindung und Waschen mit PBS werden die Antikörper nach folgenden Programm eluiert:
1. 30 min PBS
2. 60 min 3 M NaCl pH 5
3. 30 min PBS
4. 60 min 0,1 M Glycin*HCl pH 2,0
5. 30 min PBS
   Flußrate 0,25 ml/min

### Ausführungsbeispiel 3

a) Elution von anti-HSA Antikörpern (Kaninchen) aus Antikörper-Antigen-Komplexen mit HSA unter Verwendung von HSA-beschichteter Mikrotiterplatten zur Ermittlung optimaler Elutionsbedingungen für die Affinitätschromatographie (Tabelle 5)
Die 96-Well Microtiterplatten wurden mit HSA beschichtet. Jedes Well wurde mit 0,1 µg anti-HSA inkubiert. Als Detektionssystem (ELISA) diente anti-Kaninchen-IgG, konjugiert mit alkalischer Phosphatase (Substrat: 4-Nitrophenylphosphat, λ₄₀₅ ₙₘ).
200 µl des jeweiligen Elutionspuffers wurden in die Wells pipettiert. Die Elution erfolgte bei Raumtemperatur im Verlaufe von einer Stunde unter ständiger Bewegung der Mikrotiterplatte. Nach gründlichen Waschen der Wells erfolgte die Detektion des anti-HSA. Für die Auswertung wurde der Mittelwert der Messungen von jeweil 8 Wells gebildet (%CV=4,4). Der Prozentsatz eluierter anti-HSA-Antikörper mit PBS wurde mit 0 % festgelegt.

**Tabelle 5:**

| Elutionspuffer zur Lösung der Ak-Ag-Komplexe Eluierte anti-HSA-AK (%) | |
|---|---|
| PBS, pH 7,3 | 0 |
| PBS + 1 % SDS, pH 7,3 | 51 |
| 0,10 M Citrat, pH 2,5^{a} | 100 |
| 0,10 M Citrat, pH 3,0^{a} | 73 |
| 0,10 M Citrat, pH 3,5^{a} | 27 |
| 0,10 M Citrat, pH 4,0^{a} | 8 |
| 0,05 M Citrat, pH 2,5^{a} | 100 |
| 0,10 M Citrat/Phosphat, pH 7,3 | 0 |
| 0,10 M Citrat/Phosphat, pH 6,0 | 0 |
| 0,10 M Citrat/Phosphat, pH 5,0 | 0 |
| 0,10 M Citrat/Phosphat, pH 4,0 | 2 |
| 3,00 M KSCN, pH 7,3 | 41 |
| 3,00 M NaCl, pH 5,0^{a} | 0 |
| 3,00 M Guanidin*HCl, pH 7,3 | 66 |
| 4,00 M Guanidin*HCl, pH 7,3 | 90 |
| 6,00 M Harnstoff, pH 7,3 | 7 |
| 0,10 M Borat, pH 11,0 | 35 |
| 0,10 M Phosphat, pH 11,5 | 49 |
| 0,10 M Phosphat, pH 12 | 91 |

| | |
|---|---|
| ^{a} mit 0,05 % Tween 20 | |

b) Immobilisierung von anti-HSA-HSA an Basen-katalysierte, aktivierte ONB-Carbonat-Sepharose 6FF in Kopplungsmedien, die als Elutionsmedien für Immunaffinitätschromatographie genutzt werden (Tabelle 6)

Die mit dem zu koppelnden Protein versetzten und filtrierten (0,45 µm) Kopplungsmedien wurden der feuchten aktivierten ONB-Carbonat-Sepharose 6FF zugegeben. Die Kopplung erfolgte für eine Stunde bei Raumtemperatur unter leichter Bewegung. Danach wurde für eine Stunde bei Raumtemperatur mit Ethanolamin in Boratpuffer (pH 8,1) blockiert. Die ONB-Carbonatgruppen wurden spektro-photometrisch (λₘₐₓ: ca. 267 nm) bestimmt.

0,5 M Phosphatpuffer, pH 7,3 diente zur Bestimmung des Referenzwertes für eine maximale Immobilisierung.

Das Waschen erfolgte wie in Beispiel 2.

Die anti-HSA-Antikörper wurden in einem Vorversuch mittels Affinitätschromatographie (0,05 - 0,1 M Citrat, pH 2,0) gewonnen, mit 0,5 M K₂HPO₄ neutralisiert, bei - 20 °C aufbewahrt und nach dem Auftauen für die Kopplung mit verdünnter HCl auf pH 3,0 bzw. 4,0 eingestellt.

Für die Kopplung des Ag-Ak-Gemisches wurde das HSA in PBS gelöst, mit verdünnter HCl auf den entsprechenden pH-Wert eingestellt und der Antikörperlösung zugesetzt.

Die Proteinbestimmung im Kopplungspuffer erfolgte spektrophotometrisch OD₂₈₀ nm (Antikörper E^{0.1%} = 1,38 und HSA E^{0,1%} = 0,67). Die immobilisierten Proteine wurden nach Behandlung der Gele mit 1 N NaOH im Überstand nach Lowry bestimmt.
Kopplungsergebnis (%) ist die relative Menge immobilisierten Proteins im Bezug zur angebotenen Proteinmenge.

### Ausführungsbeispiel 4

Nachweis der biologischen Aktivität (Bindungsfähigkeit) von Antigenen und Antikörpern am Modell von anti-HSA/HSA nach Immobilisierung unter Spaltbedingungen für Immunkomplexe (Tabelle 7)

An Basen-katalysierte, aktivierte ONB-Carbonat-Sepharose 6FF wurde unter Elutionsbedingungen (pH 3,0; pH 4,0; 4 M Guanidin*HCl) wie in den Ausführungsbeispielen 1 und 3b) beschrieben mit anti-HSA/HSA gekoppelt. Nach Waschen der Säule wurde HSA bzw anti-HSA im Bindungspuffer angeboten. Nach erneuten Waschen der Säule mit PBS erfolgte die Elution (pH 2,0) und photometrischen Bestimmung der Proteinkonzentration.

Mit diesen Modellversuchen wird nachgewiesen, daß sowohl Antigen (HSA) wie auch Antikörper (anti-HSA) aus Immunkomplexe (anti-HSA/HSA), die unter Spaltbedingungen an einen Träger immobilisiert werden, ihre Bindungsfähigkeit erhalten. Das aus Immunkomplexen immobolisierte HSA band stets anti-HSA, daß sich nach erneuter Elution im ELISA durch eine hohe Reaktivität zu HSA auszeichnete (Ergebnisse nicht dargestellt). Unter Voraussetzung, daß 1 Mol HSA ein Mol anti-HSA bindet, wurden aus den Immunkomplexen (Versuch 3) 0,9 mg HSA/ml Gel immobilisiert, die 2,1 mg anti-HSA banden. Ähnliche Ergebnisse ließen sich mit den Versuchen 5, 7, 13 und 16 reproduzieren. Das aus den Immunkomplexen immobilisierte anti-HSA (Kaninchen-IgG) wird in diesem Modell auch unter Standardkopplungsbedingungen zwar effizient immobilisiert, der Erfolg kann mit anti-Kaninchen-IgG nachgewiesen werden, aber offensichtlich erfolgt die Kopplung hier in einem Molekülbereich, der zu sterischen Behinderungen für die Bindung des HSA führt. Die Antikörper selbst erhalten ihre biologische Aktivität.

### Ausführungsbeispiel 5

Affinitätschromatographie des Plasmas eines Lupus erythematodes-Patienten (gleichzeitig Standardverfahren)
Das Plasma eines unter Lupus erythematodes leidenden Patienten stand zur Verfügung. Für die Gewinnung der gesamten γ-Globuline wurde eine Protein A-Säule (PHARMACIA) verwendet. Die Immobilisierung der Antikörper erfolgte über ONB-Carbonat aktivierte Sepharose 6 FF (20 µmol/ml).

### Pufferlösungen für die Immunadsorption

| | | |
|---|---|---|
| Puffer PA pH 7,0 | 1000 ml | Der pH Wert wird mit HCl oder NaOH eingestellt |
| Trinatriumcitrat | 3,30 g | |
| Natriumacetat x 3H₂O | 5,45 g | |
| Natriumchlorid | 4,90 g | |
| Dinatriumhydrogenphosphat | 2,91 g | |
| Kaliumdihydrogenphosphat | 0,26 g | |
| Eluant PA pH 2,2 | 1000 ml | Der pH Wert wird mit HCl oder NaOH eingestellt |
| Zitronensäure x H₂O | 6,12 g | |
| Natriumchlorid | 9,00 g | |
| Waschpuffer 3 M NaCl pH 7,0 | 1000 ml | Der pH Wert wird mit HCl oder NaOH eingestellt |
| Trinatriumcitrat | 3,30 g | |
| Natriumacetat x 3H₂O | 5,45 g | |
| Natriumchlorid | 175,00 g | |
| Dinatriumhydrogenphosphat | 2,91 g | |
| Kaliumdihydrogenphosphat | 0,26 g | |
| Tween 20 | 0,50 g | |
| Zitratpuffer 0,1 M pH 2,2 | 250 ml | Der pH Wert wird mit NaOH eingestellt |
| Zitronensäure | 5,25 g | |

### Methoden:

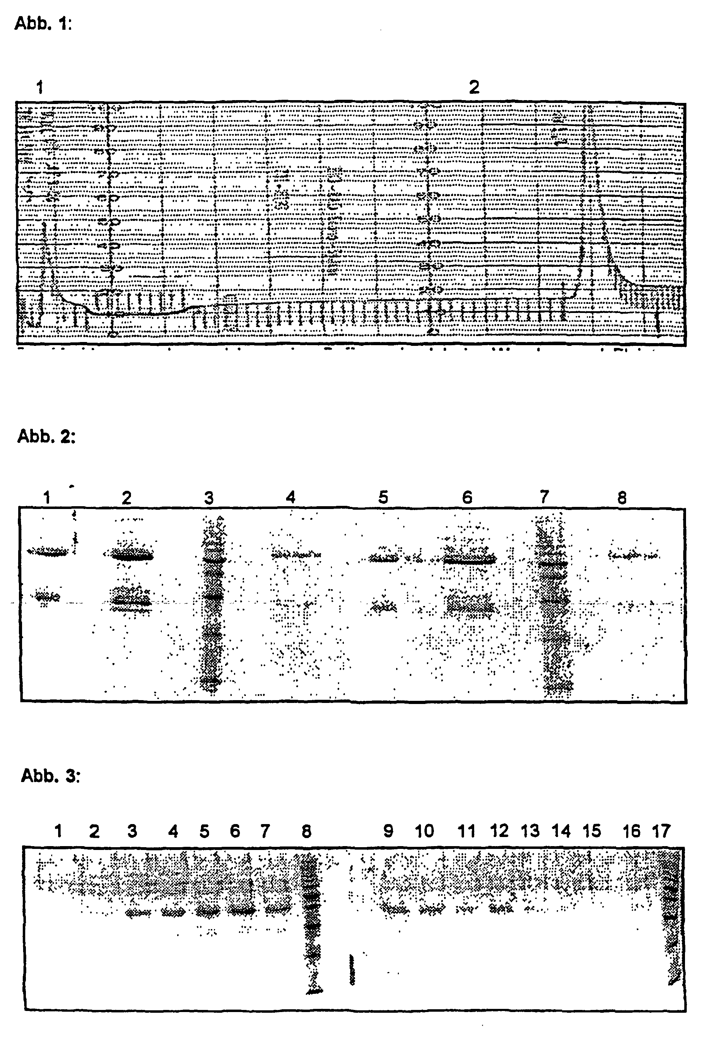

Eine Protein A-gekoppelte Säule (5 ml Gel, Pharmacia) wurde mit Puffer PA äquilibriert. 20 ml hochtourig zentrifugierten, frischen Plasmas wurden 1:2 mit Puffer PA gemischt und aufgetragen. Zur Chromatographie bedienten wir uns eines Econo-Systems (BIORAD). Das Plasma wurde nach verlassen der Säule erneut aufgetragen, um eine vollständige Adsorption zu erreichen. Intensives Waschen mit 5 Säulenvolumen PA war notwendig, um nicht adsorbiertes Material zu entfernen. Unspezifisch gebundene Proteine beseitigte Waschpuffer, der 3 M MaCl enthielt. Mit 0,1 M Citratpuffer, 0,05 % TWEEN 20 pH 2,2 eluierten die Immunglobuline und Proteine aus den Immunkomplexen als scharfer Peak. Das Volumen des Eluates betrug 6,5 ml. Die Proteinkonzentration wurde über die UV-Adsorption bei λ_{280 nm} mit 17,6 mg/ml bestimmt. Sofort nach der Elution erfolgte die Kopplung der getrennt vorliegenden IgG und Proteine an die ONB-Carbonat-aktivierte Sepharose. Dazu wurden 6 ml des nach Herstellervorschrift vorbereiteten, abgesaugten Gels zu dem Eluat gegeben und 1h geschüttelt. Das Eluat hat wegen der Pufferwirkung der darin gelösten Proteine einen pH-Wert zwischen 3 und 4. Freie Bindungen mußten mit 1 M Ethanolamin in 0.1 M Boratpuffer pH 8,0 abgesättigt werden. Durch Vergleich der Proteinkonzentrationen in den vereinigten Waschlösungen mit dem Protein A-Eluat stellten wir eine Bindungseffizienz von 57% fest. Nach gründlichem Waschen steht das Gel als Träger für affinitätschromatographische Experimente zur Verfügung.
Dazu wurden 40 ml Patientenplasma zentrifugiert, 1:2 mit PA verdünnt und 2 mal über die Säule gegeben. Ungebundenes bzw. unspezifisch gebundenes Material wurde durch aufeinanderfolgendes Waschen mit je 10 Säulenvolumen PA, Waschpuffer und PA entfernt. Mit 0,1 M Citratpuffer pH 2,2 wurde das spezifisch gebundene Protein eluiert (Abbildung 1).

Die Plasmaproteine und die chromatographisch gewonnenen Fraktionen wurden in der SDS-Polyacrylamidgelelektrophorese (SDS-PAGE) nach Standardverfahren analysiert (Miniprotean II, BioRad). Die Gele waren Gradientengele von 10 - 25 % Monomerkonzentration. Die Färbung erfolgte mit Coomassie Brillant Blue R-250 (Abbildungen 2 und 3).

Aus der Abbildung 2 ist ersichtlich, daß im Protein A-Eluat neben Antiklörpern noch weiter Proteine vorhanden sind. Nach deren Immobilisierung sind sie in der Lage, die entsprechenden Reaktionspartner aus dem Patientenplasma spezifisch zu binden. Im Elutionspeak 2 (0,1 M Citratpuffer, pH 2,2) nach der Affinitätschromatographie (Abb. 1) konnten mehrere Proteine in der PAGE identifiziert werden. Neben Immunglobulinen und mehreren höhermolekularen Proteinen, sind 3 Proteine entscheidend angereichert, die nach PAGE des Protein A-Eluates nur als kaum sichtbare Banden nachweisbar sind (Abb. 3). Sie haben eine relative Molmasse um 40 kD.

### Ausführungsbeispiel 6

Affinitätschromatographie des Plasmas eines Multible Sclerose-Patienten

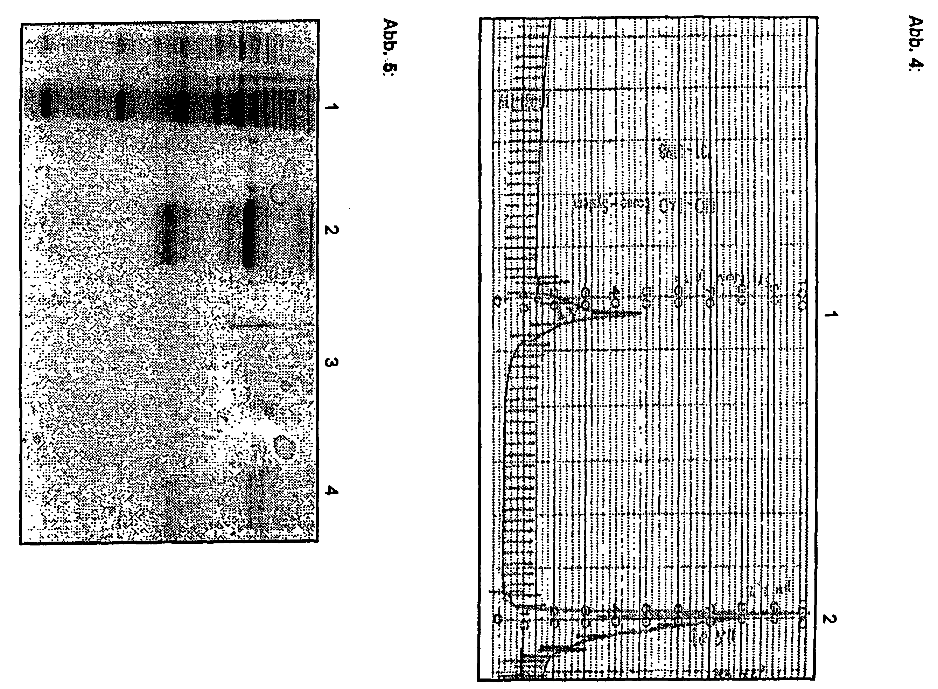

Plasmaaufarbeitung, Protein A-Elution und Kopplung der Eluatproteine geschah analog zum Ausführungsbeispiel5. Nach Abwaschen adsorptiv gebundener Proteine vom Immunadsorber (Peak 1, Abb. 4) wird mit dem Elutionspuffer spezifisch gebundenes Protein von der Matrix abgelöst, daß sich nach PAGE (Abb. 5) als ein Proteingemisch erweist, in dem hauptsächlich Immunglobuline vorhanden sind.

### Ausführungsbeispiel 7

Affinitätschromatografie des Plasmas eines Rheumatoide Arthritis-Patienten

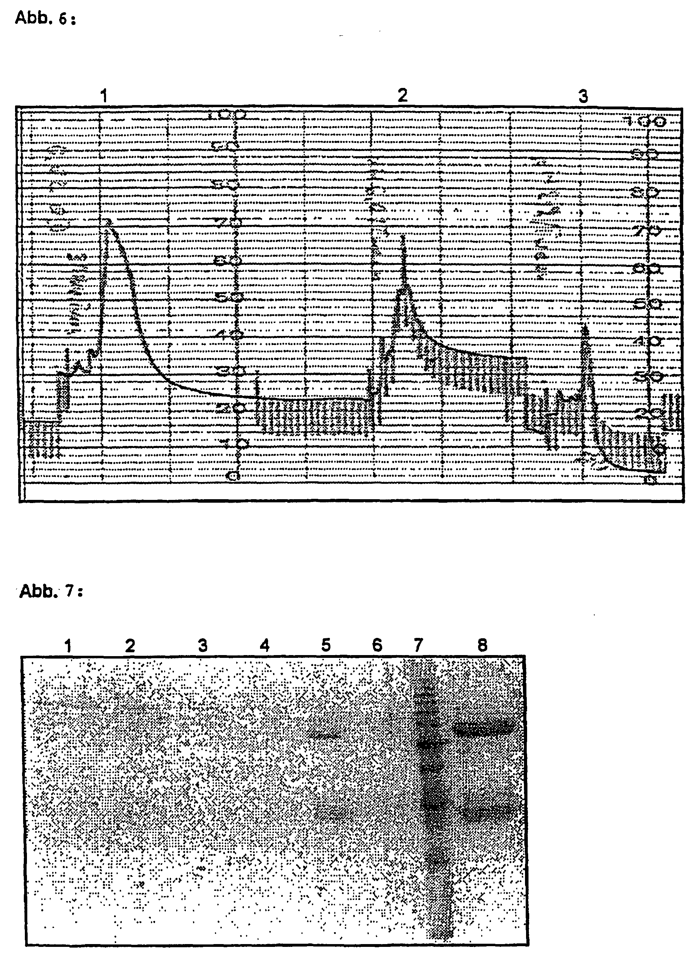

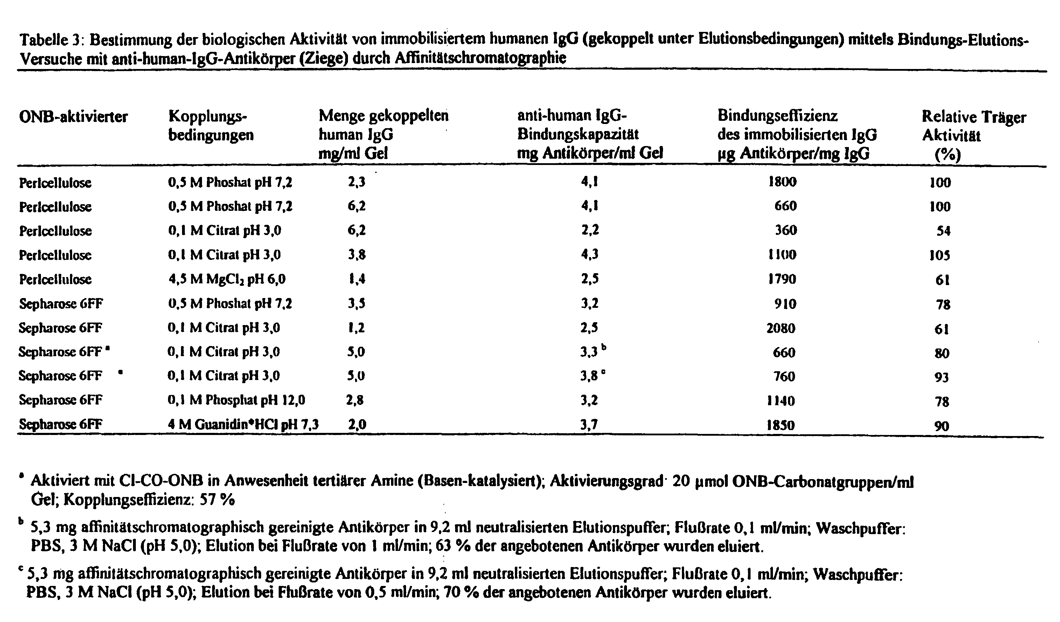

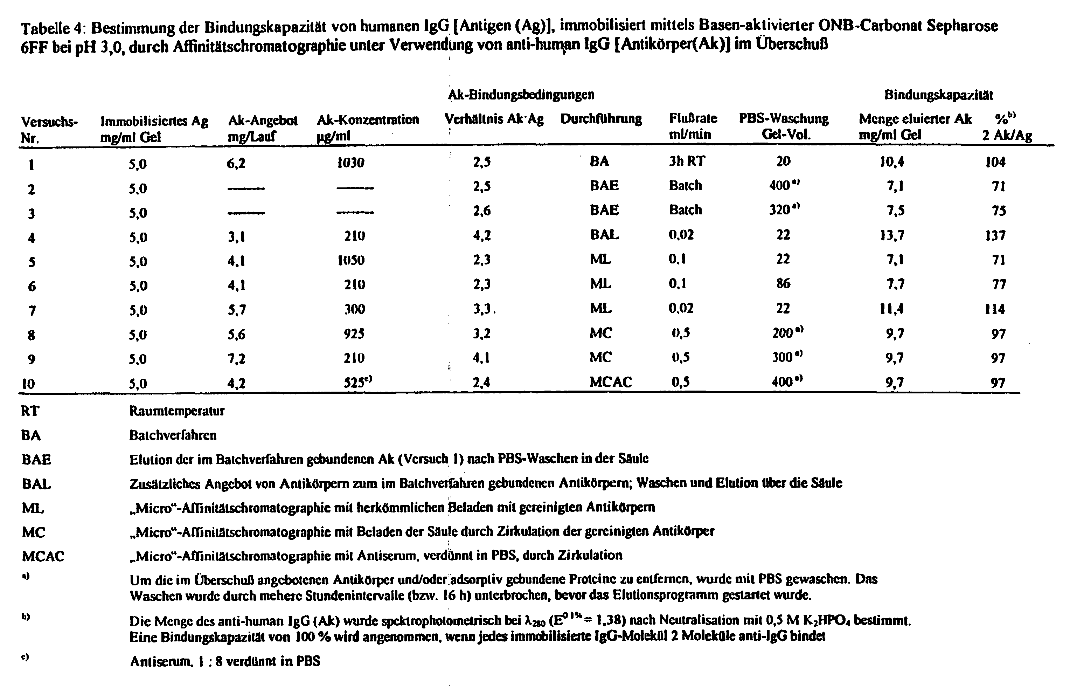

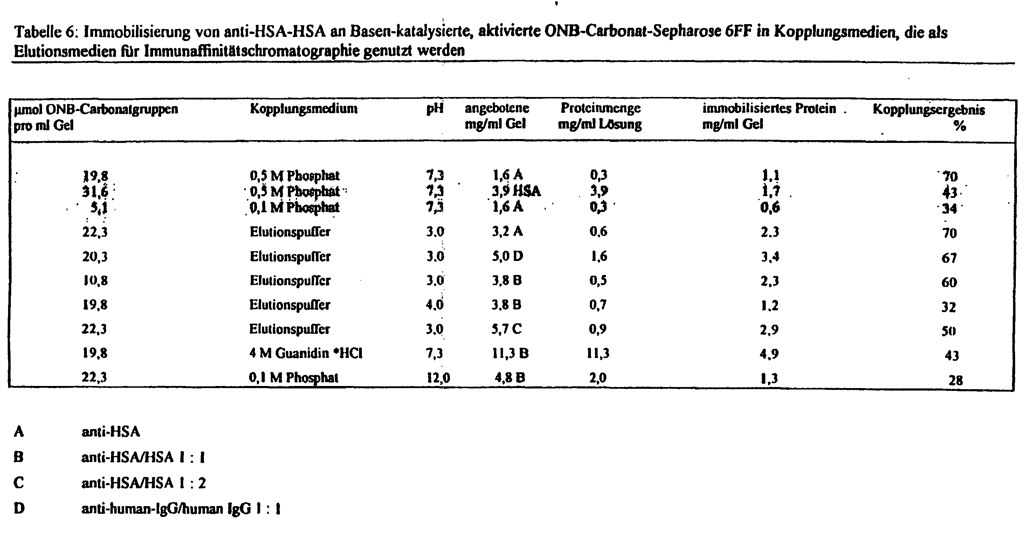

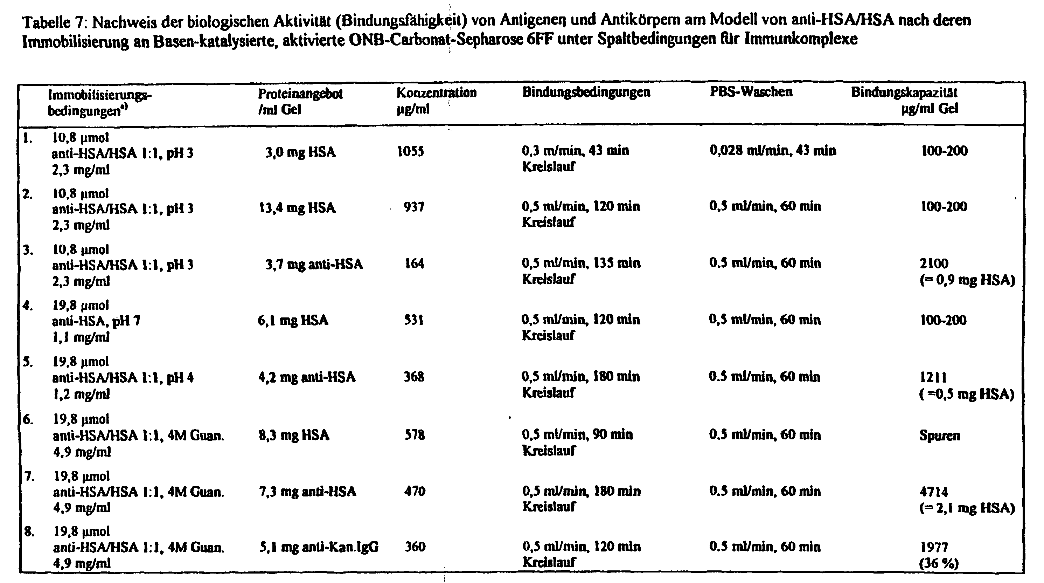

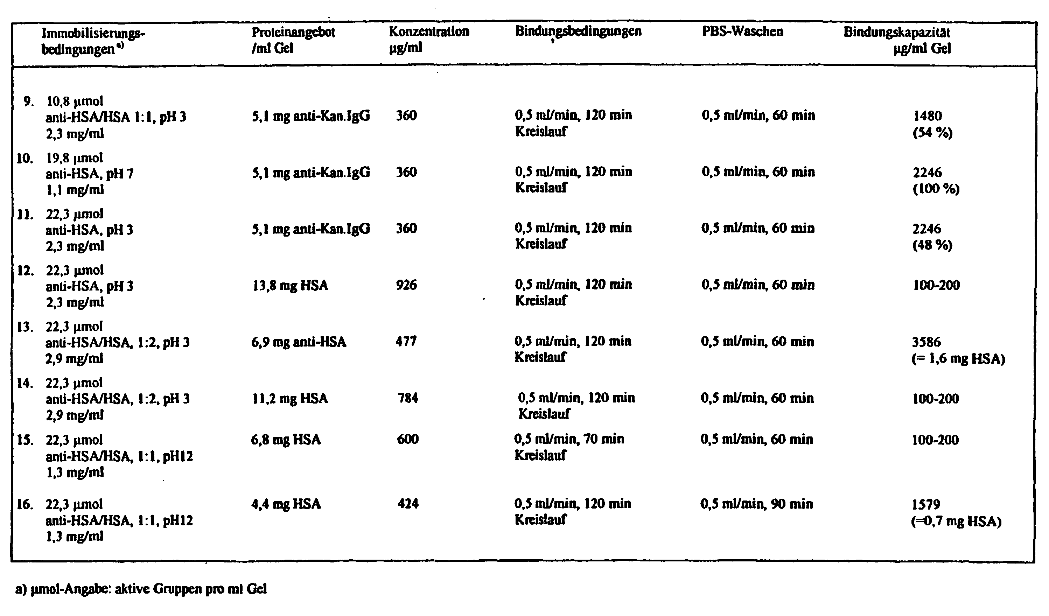

Plasmaaufarbeitung, Protein A-Elution und Kopplung der Eluatproteine geschah analog zum Ausführungsbeispiel 5. Nach Abwaschen adsorptiv gebundener Proteine vom Immunadsorber (Peak 1, Abb. 6) wird mit dem Elutionspuffer spezifisch gebundenes Protein von der Matrix abgelöst, daß sich nach PAGE (Abb. 7) als ein Proteingemisch erweist, in dem hauptsächlich Immunglobuline vorhanden sind.

Legende zu den Abbildungen 1 bis 6

### Abb. 1:

Elutionsprofil des Patientenplasmas nach Adsorption an die mit homologen Antikörpern und Antigenen gemäß Standardverfahren gekoppelte Sepharose 6FF
Die Markierungen kennzeichnen den Pufferwechsel beim Waschen und Eluieren (die Beladung der Säule wird nicht angezeigt): Nr. 1 - Waschpuffer, Nr. 2 - 0,1 M Citratpuffer pH 2,2

### Abb. 2:

PAGE des Eluates von der Protein A-Säule vor und nach der Kopplung an die ONB-Carbonat-aktivierte Sepharose
Nr- 1/2 = 5 bzw. 10 µl Material vor Kopplung, Nr. 3 = 10 kD-Leiter, Nr. 4 = γ-Globulinstandard, Nr. 5-8 wie 1-4, aber nach Kopplung (d.h. nicht gebundenes Material)

### Abb. 3:

PAGE des Eluates, das bei pH 2,2 von der Immunosorbent-Säule nach Passieren des homologen Plasmas gewonnen wurde
Nr. 1-7/9-15 = Peakfraktionen, Nr. 8/17 = 10 kD Protein-marker, Nr. 16 = Nachspülung

### Abb. 4:

Elutionsprofil des Patientenplasmas nach Adsorption an die mit homologen Antikörpern und Antigenen gemäß Standardverfahren gekoppelte Sepharose 6FF
Die Markierungen kennzeichnen den Pufferwechsel beim Waschen und Eluieren (die Beladung der Säule wird nicht angezeigt): Nr. 1 - Waschpuffer, Nr. 2 - 0,1 M Citratpuffer pH 2,2

### Abb. 5:

PAGE des Eluates, das bei pH 2,2 von der Immunosorbent-Säule nach Passieren des homologen Plasmas gewonnen wurde
Nr. 1 = 10 kD Standards, Nr. 2 = γ-Globulinstandard, Nr. 3 = Peakfraktion (Peak 2 Abb. 4), Nr. 4 = Peakfraktion nach Einengen über Amicon centrifree®

### Abb. 6:

Elutionsprofil des Patientenplasmas nach Adsorption an die mit homologen Antikörpern und Antigenen gemäß Standardverfahren gekoppelte Sepharose 6FF
Die markierungen kennzeichnen den Pufferwechsel beim Waschen und Eluieren (die Beladung der Säule wird nicht gezeigt): Nr. 1 - Waschpuffer, Nr. 2 - 0,1 M Citratpuffer pH 6,0/Tween, Nr. 3 - 0,1 M Citratpuffer pH 2,2

### Abb. 7:

PAGE des Eluates, das bei pH 2,2 von der Immunosorbent-Säule nach Passieren des homologen Plasmas gewonnen wurde

Nr. 1-4 = Proteine aus dem Waschpuffer, Nr. 5/6 = Peakfraktionen (Peak 3 Abb. 6), Nr. 7 = 10 kD Strandards, Nr. 8 = γ-Globulinstandard

## Patentansprüche

1. Patientenspezifische Immunadsorber, bestehend aus getrennten Antigenen und/oder Antikörpern aus Immunkomplexen pathologisch relevanter Immunfaktoren von Patienten, kovalent gebunden an aktivierte feste Trägermaterialien.

2. Patientenspezifische Immunadsorber nach Anspruch 1, enthaltend getrennte Antigene und/oder Antikörper von Patienten, die an Erkrankungen leiden, die durch Dysregulation des Immunsystems hervorgerufen oder aufrechterhalten werden.

3. Patientenspezifische Immunadsorber nach Anspruch 1 und 2, enthaltend getrennte Antigene und/oder Antikörper von Patienten, die an Autoimmunkrankheiten oder immunpathologischen Reaktionslagen, wie Rheumatoide Arthritis, rapid-progressive Glomerulonephritis, systemischer Lupus erythematodes, Multiple Sklerose, Anti-Phosphoid-Syndrom, Vaskulitiden, histoinkompatible Transplantatempfänger, Polymyositis, neurologische Autoimmunkrankheiten oder immunpathologische Dysregulationen in Folge von Infektionskrankheiten leiden.

4. Patientenspezifische Immunadsorber nach Anspruch 1 - 3, enthaltend getrennte Antigene und/oder Antikörper von Patienten, die an Rheumatoide Arthritis, Lupus erythematodes oder Multiple Sklerose leiden.

5. Patientenspezifische Immunadsorber nach Anspruch 1 - 4, gebunden an bioverträgliche Stoffe, die ausreichend viele Immunkomplexbestandteile auf ihrer Oberfläche kovalent binden können.

6. Patientenspezifische Immunadsocber nach Anspruch 1 - 5 gebunden an Sepharose oder Perlcellulose.

7. Verfahren zur Herstellung patientenspezifischer Immunadsorber gemäß Ansprüchen 1 - 6, **gekennzeichnet durch** Isolierung von Immunkomplexen aus Blut oder anderen Körperflüssigkeiten, wie z.B. Liquor cerebrospinalis, von Patienten und Spaltung in die aktiven Bestandteile Antikörper und Antigene sowie kovalente Kopplung dieser Bestandteile an feste Trägermaterialien.

8. Verfahren nach Anspruch 7, **dadurch gekennzeichnet, daß** die Immunkomplexe im sauren bzw. alkalischen Milieu, vorzugsweise in pH-Bereichen von 2 - 5 bzw. 10 - 12, mit oder ohne Zusatz von Salzen wie NaCl, MgCl₂, LiCl oder Harnstoff oder Guanidinhydrochlorid in ihre Einzelbestandteile zerlegt und, ggf. nach Fraktionierung und ggf. nach Zusatz von Salzen wie NaCl, MgCl₂, LiCl oder Harnstoff oder Guanidinhydrochlorid, die geeignet sind, ab einer bestimmten Konzentration die Reaktionspartner im dissoziierten Zustand zu halten, in einem pH-Bereich von 2 - 12 mittels an sich bekannter Methode an die festen Materialien kovalent gekoppelt werden.

9. Verwendung der patientenspezifischen Immunadsorber gemäß Ansprüchen 1 - 6 für die extrakorporale Apherese.

10. Verwendung der patientenspezifischen Immunadsorber gemäß Ansprüchen 1 - 6 für die Gewinnung und Weiterbearbeitung der immunpathologisch bedeutsamen körpereigenen Stoffen.

## Claims

1. Patient-specific immunoadsorbers, comprising separate antigens and/or antibodies from immunocomplexes of pathologically relevant immune factors of patients, covalently bound to activated firm carrier materials.

2. Patient-specific immunoadsorbers according to Claim 1, containing separate antigens and/or antibodies from patients suffering from diseases caused or maintained by dysregulation of the immune system.

3. Patient-specific immunoadsorbers according to Claims 1 and 2 containing separate antigens and/or antibodies from patients suffering from auto-immune diseases or immunopathological reaction situations such as rheumatoid arthritis, rapidly progressive glomerulonephritis, systemic lupus erythematodes, multiple sclerosis, anti-phosphoid syndrome, vasculitides, histoin-compatible transplant recipients polymyositis, neurological auto-immune diseases or immunopathological dysregulations as a result of infectious diseases.

4. Patient-specific immunoadsorbers according to Claims 1 - 3, containing separate antigens and/or antibodies from patients suffering from rheumatoid arthritis, lupus erythematodes or multiple sclerosis.

5. Patient-specific immunoadsorbers according to Claims 1 - 4 bound to bio-compatible materials which can covalently bind sufficient quantities of immunocomplex components on their surfaces.

6. Patient-specific immunoadsorbers according to Claims 1 - 5 bound to sepharosis or pearl cellulose.

7. Method for the production of patient-specific immunoadsorbers according to Claims 1 - 6, wherein there exists isolation of immunocomplexes from blood or other body fluids, e.g. liquor cerebrospinalis, from patients and splitting into the active component parts of antibodies and antigens as well as covalent coupling of these components to firm carrier materials.

8. Method according to Claim 7 wherein the immune complexes are split up into their individual components in an acid or alkaline milieu, as the case may be, preferably in pH ranges of 2 - 5 or 10 - 12 respectively, with or without addition of salts such as NaCl, MgCl₂, LiCl or urea or guanidine hydrochloride and, if need be, following fractioning and, if need be, following addition of salts such as NaCl, MgCl₂, LiCl or urea or guanidine hydrochloride, which are suited to keep the reaction partners in a dissociated condition from a certain concentration onwards, are covalently coupled to the firm materials in a pH range of 2 - 12 by means of a method already known per se.

9. Use of the patient-specific immunoadsorbers according to Claims 1 - 6 for extra-corporal apheresis.

10. Use of the patient-specific immunoadsorbers according to Claims 1 - 6 to obtain and further process the body-inherent materials of immunopathological importance.

## Revendications

1. Adsorbant immunitaire spécifique au patient, se composant d'antigènes et/ou d'anticorps séparés de complexes immunitaires de facteurs immunitaires d'importance pathologique de patients, liés en covalence à des matériaux supports solides activés.

2. Adsorbant immunitaire spécifique au patient conformément à la revendication 1, contenant des antigènes et/ou des anticorps séparés de patients qui souffrent de maladies provoquées ou entretenues par la dérégulation du système immunitaire.

3. Adsorbant immunitaire spécifique au patient conformément à la revendication 1 et 2, contenant des antigènes et/ou des anticorps séparés de patients qui souffrent de maladie auto-immunes ou de situations réactives d'immunopathologie, telles que l'arthrite rhumatoïde, la néphrite glomérulaire rapide et progressive, le lupus érythémateux d'aspect systémique, la-multiple sclérose, le syndrome anti-phospholipide, les vasculites, les receveurs de greffes à incompatibilité histologique, les polymyosites, les maladies neurologiques auto-immunes ou les dérégulations d'immunopathologie par suite de maladies infectieuses.

4. Adsorbant immunitaire spécifique au patient conformément à la revendication 1 à 3, contenant des antigènes et/ou des anticorps séparés de patients qui souffrent d'arthrite rhumatoïde, du lupus érythémateux ou de multiple sclérose.

5. Adsorbant immunitaire spécifique au patient conformément à la revendication 1 à 4, lié à des substances biocompatibles qui peuvent lier en covalence un nombre suffisamment grand d'éléments du complexe immunitaire sur leur surface.

6. Adsorbant immunitaire spécifique au patient conformément à la revendication 1 à 5, lié à de la sépharose ou à de la cellulose en perles.

7. Procédé permettant la fabrication d'adsorbant immunitaire spécifique au patient conformément aux revendications 1 à 6, se caractérisant par l'isolation de complexes immunitaires issus de sang ou d'autres liquides corporels, tels que la liqueur cérébrospinale, de patients et par la scission en éléments actifs anticorps et antigènes ainsi que par copulation covalente de ces éléments à des matériaux supports solides.

8. Procédé conformément à la revendication 7, se caractérisant par le fait que les complexes immunitaires sont décomposés en leurs différents composants dans un milieu acide voire alcalin, de préférence entre un pH de 2 à 5 voire de 10 à 12, avec ou sans addition de sels tels que NaCl, MgCl₂, LiCl ou urée ou hydrochlorure de guanidine et, le cas échéant après fractionnement et le cas échéant après addition de sels tels que NaCl, MgCl₂, LiCl ou urée ou hydrochlorure de guanidine qui sont en mesure de maintenir les réactants en état dissocié à partir d'une certaine concentration, sont copulés en covalence aux matériaux solides entre un pH de 2 à 12 au moyen de la méthode connue en soi.

9. Emploi de l'adsorbant immunitaire spécifique au patient conformément aux revendications 1 à 6 pour l'aphérèse extracorporelle.

10. Emploi de l'adsorbant immunitaire spécifique au patient conformément aux revendications 1 à 6 pour l'extraction et le traitement ultérieur des substances corporelles importantes au niveau de l'immunopathologie.
